(19) **Europäisches Patentamt / European Patent Office / Office européen des brevets**

(11) **EP 4 760 726 A1**

(12) **EUROPEAN PATENT APPLICATION**
published in accordance with Art. 153(4) EPC

(43) Date of publication:
17.06.2026 Bulletin 2026/25

(21) Application number: 24870717.6

(22) Date of filing: 24.09.2024

(51) International Patent Classification (IPC):
*G16B 30/00* (2019.01)  *G16B 40/20* (2019.01)
*G06F 16/33* (2025.01)  *G06F 16/35* (2025.01)
*G06F 18/24* (2023.01)  *G06F 40/289* (2020.01)

(86) International application number:
PCT/CN2024/120794

(87) International publication number:
WO 2025/067173 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 CN 202311261520

(71) Applicant: Likang Life Sciences
Beijing 100023 (CN)

(72) Inventors:
• CHEN, Li
Beijing 100023 (CN)

• JIA, Mingming
Beijing 100023 (CN)
• LI, Qing
Beijing 100023 (CN)
• LIU, Sanyang
Beijing 100023 (CN)
• YAO, Mingyue
Beijing 100023 (CN)

(74) Representative: Huang, Liwei
Cäcilienstraße 12
40597 Düsseldorf (DE)

Remarks:
The complete document including Reference
Table(s) and the Sequence Listing(s) can be
downloaded from the EPO website

(54) **METHOD FOR IDENTIFYING PEPTIDE FRAGMENT BOUND TO CLASS MHC-I/HLA-I AND RECOGNIZED BY TCR, DEVICE, AND STORAGE MEDIUM**

(57)    The present invention discloses a method, a device, and a storage medium for identifying peptides capable of binding to MHC-I/HLA-I and being recognized by TCR. The method includes: acquiring protein sequences, MHC-I/HLA-I sequences, and peptide sequences; training a protein sequence tokenizer by using the protein sequences; training a protein representation model by using the protein sequences tokenized by the protein sequence tokenizer to obtain a trained protein representation pre-trained model; fine-tuning the trained protein representation pre-trained model by using the MHC-I/HLA-I sequences and the peptide sequences tokenized by the protein sequence tokenizer, to obtain a binary classification model; and identifying target wild-type/mutant peptides and classifying mutant peptides by using the binary classification model, to obtain candidate neoantigen mutant peptides that are positively correlated with TCR recognition.

FIG. 1

**Description**

TECHNICAL FIELD

The present invention relates to the field of vaccines, and in particular to a method, a device, and a storage medium for identifying peptides capable of binding to MHC-I/HLA-I and being recognized by T-cell receptor (TCR).

BACKGROUND

Antigen screening technologies are mainly used to screen out antigens capable of eliciting the strongest immune responses during vaccine preparation. In the antigen screening process, it is necessary to evaluate antigens from different sources and then determine optimal combinations of antigens. Commonly used antigen screening technologies include biochip-based screening technologies and protein analysis-based screening technologies, and the like. Tumor vaccines are currently a research focus in the field of vaccines. The earliest tumor vaccines were reported in 1994, and in recent years, advances in technology have enabled tumor vaccines to achieve targeted treatment of patient-specific mutant antigens. By introducing peptides or mRNA of tumor-associated antigens or tumor-specific antigens into patients, the immune system of the patients can be activated. Breakthroughs in personalized tumor vaccine technologies critically depend on the development in the detection of neoantigens and prediction technologies for the preparation of neoantigen vaccines. Predicting the binding potential between specific mutant peptides and histocompatibility complexes is an important part of the detection of neoantigens, because tumor-specific antigens must be recognized by major histocompatibility complex (MHC) and presented to the cell surface before they can be further recognized by T cell receptors and ultimately lead to tumor cell killing. Therefore, it is of great significance to predict the binding between peptides and MHC-I/HLA-I, as well as whether the resulting pMHC complexes can maintain stable binding, be presented to the cell surface and recognized by TCR.

In recent years, the field of neoantigens has developed rapidly, leading to the generation of a wide range of large-scale databases that can be used for training affinity prediction models for peptides binding to MHC-I/HLA-I. At the same time, a variety of algorithms have been applied to predict the binding potential between peptides and MHC-I/HLA-I, including traditional machine learning methods and deep learning methods with rapid development. Artificial neural networks (ANNs), convolutional neural networks (CNNs), stabilized matrix method (SMM), random forest methods, and other approaches have all been used to construct affinity prediction models. Although these algorithms have been reported to exhibit good performance when tested on test datasets, their performance is often only moderate when tested on completely independent datasets. The affinity prediction for peptide binding to MHC-I/HLA-I still suffers from relatively high error rates, particularly high false positive rates, which can have serious consequences in clinical applications. The causes of these issues mainly include the following aspects: first, it is difficult to obtain comprehensive and sufficiently large datasets for model training, and limited datasets make it challenging to learn more informative features; second, conventional algorithms applied to prediction models rarely take into account intrinsic features of protein sequences and sequence characteristics that may play important roles during protein-protein sequence interactions; third, both the representation of the full-length sequence of MHC-I/HLA-I and the representation of the pseudo-sequence limit the ability of models to specifically learn features of key sites involved in peptide binding, thereby adversely affecting model performance; fourth, most existing methods focus only on pMHC presentation, while features of mutant peptides, such as binding positions and the types of amino acid residues, are considered in isolation without further accounting for pMHC binding stability, pMHC presentation capability, and central tolerance mechanisms that play important roles in TCR (T-cell receptor) recognition, thus affecting the accurate selection of neoantigen mutant peptides.

SUMMARY

To address the above-mentioned problems, the present invention provides a method, a device, and a storage medium for identifying peptides capable of binding to MHC-I/HLA-I and being recognized by TCR. The method enables more targeted identification of key features involved in recognition, transport, presentation, and TCR recognition of tumor-specific peptides, and addresses the limitation of insufficient data volume for model construction by applying novel modeling strategies for model training, feature extraction, and classification, thereby improving the accuracy of identifying peptides capable of binding to MHC-I/HLA-I and as well as the accuracy of neoantigen identification.

Embodiments of the present invention provide a method for identifying peptides capable of binding to MHC-I/HLA-I and being recognized by TCR, including following operations:

acquiring protein sequences, MHC-I/HLA-I sequences, and peptide sequences;
training a protein sequence tokenizer by using the protein sequences;
training a protein representation model by using the protein sequences tokenized by the protein sequence tokenizer to obtain a trained protein representation pre-trained model;

fine-tuning the trained protein representation pre-trained model by concatenating the MHC-I/HLA-I sequences and the peptide sequences tokenized by the protein sequence tokenizer to obtain a binary classification model;

identifying target wild-type/mutant peptides and classifying the mutant peptides by using the binary classification model to obtain candidate neoantigen mutant peptides that are positively correlated with TCR recognition.

The binary classification model can be applied to predict peptides capable of binding to MHC-I/HLA-I, and candidate neoantigens can be predicted by applying the binary classification model in combination with the positions at which mutations occur.

Embodiments of the present invention provide a method for identifying peptides capable of binding to MHC-I/HLA-I and being recognized by TCR, including following operations:

acquiring protein sequences, MHC-I/HLA-I sequences, and peptide sequences;

training a protein sequence tokenizer by using the protein sequences;

training a protein representation model by using the protein sequences tokenized by the protein sequence tokenizer to obtain a trained protein representation pre-trained model;

fine-tuning the trained protein representation pre-trained model by concatenating the MHC-I/HLA-I sequences and the peptide sequences tokenized by the protein sequence tokenizer to obtain a binary classification model; and

identifying peptides to be evaluated by using the binary classification model to obtain peptides capable of binding to MHC-I/HLA-I.

The MHC-I refers to major histocompatibility complex class I (MHC class I), the HLA- I refers to major histocompatibility complex class I (MHC class I) in humans, and the TCR refers to a T cell receptor.

Preferably, the peptide sequences include positive peptide sequences and negative peptide sequences, in which the positive peptide sequences are the sequences of peptides capable of binding to MHC- I/HLA- I and are labeled as 1, and the negative peptide sequences are peptide sequences randomly generated from protein sequences based on features of the positive peptide sequences and are labeled as 0.

Preferably, the protein sequences include protein sequences collected from a protein database. Accordingly, collected protein sequences are used to train a tokenizer for high-frequency repeated amino acid residue combination motifs, to obtain the protein sequence tokenizer.

Preferably, training the protein representation model by using the protein sequences tokenized by the protein sequence tokenizer to obtain the trained protein representation pre-trained model includes following operations:

inputting unlabeled protein sequences into the protein sequence tokenizer for tokenization, to obtain protein sequences segmented into functional amino acid residue combination motifs;

converting the protein sequences segmented into the functional amino acid residue combination motifs into numerically represented protein sequences; and

training the protein representation model by using the numerically represented protein sequences to obtain the trained protein representation pre-trained model.

Preferably, fine-tuning the trained protein representation pre-trained model by using the MHC-I/HLA-I sequences and the peptide sequence tokenized by the protein sequence tokenizer to obtain the binary classification model includes following operations:

inputting labeled MHC- I/HLA- I sequences into the protein sequence tokenizer for tokenization, to obtain MHC- I/HLA- I sequences segmented into functional amino acid residue combination motifs, and converting the MHC- I/HLA- I sequences segmented into functional amino acid residue combination motifs into numerically represented MHC- I/HLA- I sequences;

converting labeled peptide sequences into numerically represented peptide sequences;

concatenating converted sequences, and fine-tuning the trained protein representation pre-trained model by using concatenated sequences to obtain the binary classification model.

In specific embodiments, fine-tuning may include inheriting the original pre-trained model and adding a classification layer. In addition, on the basis of adding the classification layer, additional layers, such as fully connected layers, attention mechanism layers, or other suitable task layers may be further added.

In some embodiments, the concatenating is performed by direct concatenation, that is, directly concatenating the numerically represented MHC-I/HLA-I sequences with the numerically represented peptide sequences.

In another embodiments, the concatenating is performed by concatenating the numerically represented MHC-

I/HLA-I sequences and the numerically represented peptide sequences, as well as additional numerically represented features related to binding between the peptides and MHC-I/HLA-I. Those skilled in the art may select an appropriate manner for the concatenating according to the objects to be concatenated. Exemplary manners for the concatenating include directly concatenating the numerically represented sequences and related features by using torch.cat(), or performing addition, subtraction, multiplication, division, or other operations on feature tensors according to a customized concatenation manner.

In specific embodiments, the additional features related to binding between the peptides and the MHC-I/HLA-I are one or more selected from a probability of cleavage of the protein sequences, a binding stability between the MHC-I/HLA-I and the peptides, and a presentation likelihood of complexes formed by the MHC-I/HLA-I and the peptides.

The probability of cleavage of the protein sequences may be obtained by using suitable software by inputting the peptide sequences. Those skilled in the art will appreciate that any suitable software and any appropriate version thereof may be used, provided that the probability of cleavage of the protein sequences can be obtained based on the peptide sequences. For example, software such as pepsickle and iPCPS may be used for this purpose.

The binding stability between MHC-I/HLA-I and the peptides may be obtained by using suitable software by inputting the peptide sequences and the MHC-I/HLA-I sequences. Those skilled in the art will appreciate that any suitable software with appropriate version thereof may be used, provided that the binding stability between MHC-I/HLA-I and the peptides can be obtained based on the peptide sequences and the MHC-I/HLA-I sequences. For example, software such as LKMHCstablepan and TLStab may be used for this purpose.

The presentation likelihood of the complex formed by MHC-I/HLA-I and the peptides may be obtained by using suitable software by inputting the peptide sequences. Those skilled in the art will appreciate that any suitable software with appropriate version thereof may be used, provided that the presentation likelihood of the complex formed by MHC-I/HLA-I and the peptides can be obtained based on the peptide sequences and, optionally, the MHC-I/HLA-I sequences. For example, software such as DeepTAP, TAPPred, and TAPREG may be used for this purpose.

Preferably, prior to the fine-tuning, the method of the present invention further includes calculating other features related to binding between the peptides and MHC-I/HLA-I by using the protein sequences, the MHC-I/HLA-I sequences, and the peptide sequences.

Preferably, identifying target wild-type/mutant peptides by using the binary classification model includes following operations:

inputting the target wild-type/mutant peptides into the binary classification model, and outputting attention scores for each position of mutant peptides predicted as positive and attention scores of functional amino acid residue combination motifs of the MHC-I/HLA-I sequences;

determining anchor positions of binding between the MHC-I/HLA-I sequences and the positive peptides as well as key representative positions of the MHC-I/HLA-I sequences, based on the attention scores for each position of the mutant peptides predicted as positive and the attention scores of the MHC-I/HLA-I sequences motifs, and classifying positive mutant peptides based on the anchor positions of binding between the MHC-I/HLA-I sequences and the peptides and mutation positions of the peptides.

Preferably, classifying the positive mutant peptides based on the anchor positions of binding between the MHC-I/HLA-I sequences and the positive mutant peptides as well as mutation positions relative to wild-type peptides includes following operations:

classifying the positive mutant peptides based on the anchor positions of binding between the MHC-I/HLA-I sequences and the peptides in combination with the mutation positions of, and determining, based on classification results, whether the positive mutant peptides are suitable for selection as candidate neoantigen mutant peptides that are positively correlated with TCR recognition.

Preferably, determining, based on the classification results, whether the positive mutant peptides are suitable for selection as candidate neoantigen mutant peptides that are positively correlated with TCR recognition includes following operations:

determining that the peptides are selectable as neoantigen mutant peptides when the wild-type peptides exhibit weak binding to MHC-I/HLA-I and weak TCR recognition (in general, wild-type peptides are weak in TCR recognition), and the mutant peptides have mutations at anchor positions for MHC-I/HLA-I and are strong in MHC-I/HLA-I binding and TCR recognition;

determining that the peptides are selectable as neoantigen mutant peptides when the wild-type peptides exhibit weak binding to MHC-I/HLA-I and weak TCR recognition (in general, wild-type peptides exhibit weak TCR recognition), and the mutant peptides have mutations at non-anchor positions for MHC-I/HLA-I and are strong in MHC-I/HLA-I binding and TCR recognition;

determining that the peptides are selectable as neoantigen mutant peptides when the wild-type peptides exhibit strong binding to MHC-I/HLA-I and weak TCR recognition (in general, wild-type peptides exhibit weak TCR recognition), and the mutant peptides have mutations at non-anchor positions for MHC- I/HLA- I and are strong in MHC- I/HLA- I binding and TCR recognition (herein, "strong TCR recognition" indicates that a sequence exposed to TCR is changed);

determining that the peptides are not selectable as neoantigen mutant peptides when the wild-type peptides exhibit strong binding to MHC- I/HLA- I and weak TCR recognition ( in general, wild-type peptides exhibit weak TCR recognition), and the mutant peptides have mutations at anchor positions for MHC- I/HLA- I and are strong in MHC-I/HLA- I binding and weak in TCR recognition (herein, "weak TCR recognition" indicates that a sequence exposed to TCR remains unchanged).

The beneficial effects of the present invention are that, by applying deep learning algorithms based on natural language processing, we can identify the recognition, transport, and presentation of wild-type/mutant peptides with histocompatibility complex molecules MHC-I/HLA-I, recognize biological features involved in protein-protein interactions, determine anchor positions for peptide binding to MHC-I/HLA-I, construct more representative sequence characterization methods for MHC-I/HLA-I are constructed, and then introduce the influence of central tolerance mechanisms on TCR recognition to further promote the identification of tumor neoantigens.

BRIEF DESCRIPTION OF THE DRAWINGS

FIG. 1 is a flowchart of a method for identifying peptides capable of binding to MHC-I/HLA-I and being recognized by TCR according to the present invention.
FIG. 2 is a flowchart of an algorithm for identifying peptides capable of binding to MHC-I/HLA-I and being recognized by TCR according to the present invention.
FIG. 3 is a flowchart of an algorithm with additional features for identifying peptides capable of binding to MHC-I/HLA-I and being recognized by TCR according to the present invention.

DETAILED DESCRIPTION

It should be understood that the specific embodiments described herein are only for illustrating the present invention and are not intended to limit the present invention. In the following description, suffixes such as "module", "component", or "unit" used to denote elements are merely for convenience of description and do not have specific meanings. Therefore, the terms "module", "component", and "unit" may be used interchangeably.

As used herein, the term "concatenate/concatenation/concatenating" refers to a process of concatenating multiple features converted into numerical representations, thereby obtaining a concatenated sequence that can be used to fine-tune a protein representation pre-trained model.

As used herein, the term "pMHC" refers to a complex formed by binding a peptide to MHC-I/HLA-I.

As used herein, the terms "other features" and "more features" can be used interchangeably, and refer to features related to the binding of peptides and MHC-I/HLA-I calculated by using protein sequences, MHC-I/HLA-1 sequences and peptide sequences. Exemplary features include a probability of cleavage of protein sequences, the binding stability of pMHC, and the presentation capability of pMHC.

The present invention provides a deep learning algorithm for identifying whether wild-type/mutant peptides are capable of binding to major histocompatibility complex class I (MHC-I/HLA-I), being transported and presented to the cell surface, and are recognized by TCR, thereby further promoting the identification of peptides capable of binding to MHC-I/HLA-I and the selection of tumor-specific neoantigens. Specifically, the method applies natural language processing techniques to protein sequence feature discovery and extraction for constructing a protein representation pre-trained model. The method utilizes protein sequences and protein-protein interaction datasets to extract amino acid residue combination motifs and key site features that are positively correlated with peptide binding, transport, and presentation, on the basis of the protein representation pre-trained model, the method fine-tunes the protein representation pre-trained model by collecting a positive peptide sequence dataset and constructing a negative (peptide sequence) dataset, thereby obtaining a model capable of accurately inferring the binding potential of wild-type/mutant peptides to MHC-I/HLA-I. The model obtained by the above method can be applied to binding prediction for peptides of diverse types or for MHC-I/HLA-I types, provides an interpretable reflection of the biological characteristics of peptide presentation, and can be further applied to model development to obtain a prediction model with improved interpretability and accuracy compared with conventional algorithms. At the same time, the model further identifies recognition features of pMHC and TCR (T-cell receptor) following peptide binding to MHC-I/HLA-I, thereby further predicting whether a peptide has the capability to activate TCR (T-cell receptor) rather than merely binding to MHC, and ultimately enabling the selection of candidate neoantigens with higher potential.

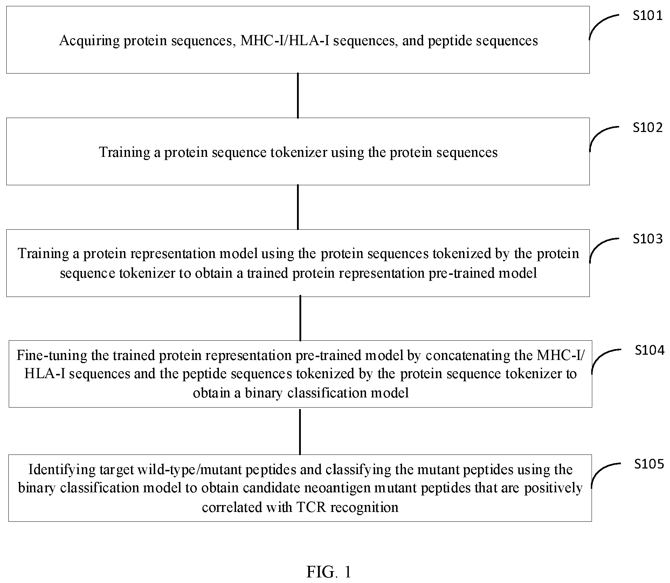

FIG. 1 is a flowchart of a method for identifying peptides capable of binding to MHC-I and being recognized by TCR provided by the present invention. As shown in FIG. 1, the method includes following operations.

At S101, protein sequences, MHC-I/HLA-I sequences, and peptide sequences are acquired.

At S102, a protein sequence tokenizer is trained by using the protein sequences.

At S103, a protein representation model is trained by using the protein sequences tokenized by the protein sequence tokenizer, to obtain a trained protein representation pre-trained model;

At S104, the trained protein representation pre-trained model is fine-tuned by using the MHC-I/HLA-1 sequences and the peptide sequences tokenized by the protein sequence tokenizer, to obtain a binary classification model.

At S105, by using the binary classification model, the target wild-type/mutant peptides are identified, and the mutant peptides are classified, so as to obtain candidate neoantigen mutant peptides that are positively correlated with TCR recognition.

Specifically, the peptide sequences contain positive peptide sequences capable of binding to MHC-I/HLA-I and are labeled as 1 and negative peptide sequences randomly generated from the protein sequences based on features of the positive peptide sequences and are labeled as 0.

The protein sequences contain protein sequences collected from a protein database. Accordingly, the collected protein sequences are used to train a tokenizer for high-frequency repeated amino acid residue combination motifs, to obtain the protein sequence tokenizer.

Further, training the protein representation model by using the protein sequences tokenized by the protein sequence tokenizer to obtain the trained protein representation pre-trained model includes: inputting unlabeled protein sequences into the protein sequence tokenizer for tokenization, to obtain protein sequences segmented into functional amino acid residue combination motifs; converting the protein sequences segmented into the functional amino acid residue combination motifs into numerically represented protein sequences; and training the protein representation model by using the numerically represented protein sequences to obtain the trained protein representation pre-trained model.

More specifically, fine-tuning the trained protein representation pre-trained model by using the MHC-I/HLA-I sequences and the peptide sequences that have been tokenized by the protein sequence tokenizer to obtain the binary classification model includes: inputting labeled MHC-I/HLA-I sequences into the protein sequence tokenizer for tokenization, to obtain MHC-I/HLA-I sequences segmented into functional amino acid residue combination motifs, and converting the MHC-I/HLA-I sequences segmented into functional amino acid residue combination motifs into numerically represented MHC-I/HLA-I sequences; converting labeled peptide sequences into numerically represented peptide sequences; concatenating the MHC-I/HLA-I sequences and the numerically represented peptide sequences to obtain concatenated sequences; and fine-tuning the trained protein representation pre-trained model by using the concatenated sequences to obtain the binary classification model. The concatenating may be performed by direct concatenation, or by concatenation with additional numerically represented features including one or more selected from a probability of cleavage of protein sequences, a binding stability between MHC-I/HLA-I and peptides, and a presentation likelihood of complexes formed by MHC-I/HLA-I and peptides.

Further, identifying target wild-type/mutant peptides by using the binary classification model includes: inputting the target wild-type/mutant peptides into the binary classification model, and outputting attention scores for each position of the mutant peptides predicted as positive, and attention scores of functional amino acid residue combination motifs of the MHC- I/HLA- I sequences; determining anchor positions of binding between the MHC-I/HLA-I sequences and the positive peptides as well as key representative positions of the MHC-I/HLA-I sequences based on the attention scores for each position of the mutant peptides predicted as positive and the attention scores of the MHC- I/HLA- I sequences motifs; and classifying the positive mutant peptides based on the anchor positions of binding between the MHC-I/HLA-I sequences and the peptides and mutation positions of the peptides.

Further, classifying the positive mutant peptides based on the anchor positions of binding between the MHC-I/HLA- I sequences and the positive mutant peptides as well as mutation positions relative to wild-type peptides includes: classifying the positive mutant peptides based on the anchor positions of binding between the MHC- I/HLA- I sequences and the peptides in combination with positions of mutations, and determining, based on classification results, whether the positive mutant peptides are suitable for selection as candidate neoantigen mutant peptides that are positively correlated with TCR recognition.

More specifically, determining, based on the classification results, whether the positive mutant peptides are suitable for selection as candidate neoantigen mutant peptides that are positively correlated with TCR recognition includes: determining that the peptides are selectable as neoantigen mutant peptides when the wild-type peptides exhibit weak binding to MHC- I/HLA- I and weak TCR recognition, and the mutant peptides have mutations at anchor positions for MHC- I/HLA- I and are strong in MHC- I/HLA- I binding and TCR recognition; determining that the peptides are selectable as neoantigen mutant peptides when the wild-type peptides exhibit weak binding to MHC- I/HLA- I and weak TCR recognition, and the mutant peptides have mutations at non-anchor positions for MHC- I/HLA- I and are strong in MHC-I/HLA- I binding and TCR recognition; determining that the peptides are selectable as neoantigen mutant peptides when the wild-type peptides exhibits strong binding to MHC- I/HLA- I and weak TCR recognition, and the mutant peptides have mutations at non-anchor positions for MHC-I/HLA-I and are strong in MHC- I/HLA- I binding and TCR recognition; determining that the peptides are not selectable as neoantigen mutant peptides when the wild-type peptides exhibit strong binding to MHC- I/HLA- I and weak TCR recognition, and the mutant peptides have mutations at anchor positions for MHC-I/HLA- I and are strong in MHC- I/HLA- I binding and weak in TCR recognition.

An electronic device is provided by embodiments of the present application. The electronic device includes a memory, a processor, and a computer program stored in the memory and configured to be executed by the processor to implement a method, a device, and a storage medium for identifying peptides capable of binding to MHC-I/HLA-I and being recognized by TCR.

A computer-readable storage medium on which a computer program is stored is provided by embodiments of the present application. The computer program is executed by a processor to implement a method for identifying peptides capable of binding to MHC-I/HLA-I and being recognized by TCR.

In summary, the present invention provides a method for inferring the binding potential of peptides to MHC-I/HLA-I, by obtaining biological features from protein sequences and applying them to peptides and MHC-I/HLA-I binding prediction models with interpretability and accuracy based on a pre-trained model. Specifically, the method includes followings operations. The protein sequence tokenizer and the protein representation pre-trained model are constructed by using lots of unlabeled protein data. Labeled training, validation, and test datasets including positive and negative datasets are created. The positive dataset includes positive peptide sequences obtained from databases through mass spectrometry or in vitro binding experiments and corresponding MHC-I/HLA-I subtypes; and the negative sequences are randomly generated from protein sequences according to length features of the positive peptide sequences. Negative peptides do not include peptides that are present in the positive peptides. MHC-I/HLA-I subtypes are tokenized by using the protein sequence tokenizer, and the MHC-I/HLA-I sequences and peptide sequences are converted into numerically represented sequences. A binary classification model is constructed by fine-tuning the protein representation pre-trained model, and supervised learning is performed on the generated numerically represented data, thereby further improving model performance for the task. The binary classification model is further applied to predict attention scores of positive peptides and MHC-I/HLA-I sequence motifs, and then based on the predicted attention scores, the manner for representing MHC-I/HLA-I is obtained and anchor positions for peptide and MHC-I/HLA-I binding is inferred. At a deeper sense, the present invention provides a method capable of inferring whether peptides are capable of binding to MHC-I/HLA-I and activate TCR responses to elicit immune responses. For such method, more accurate neoantigens are obtained by utilizing anchor positions for peptide-MHC-I/HLA-I binding, classifying wild-type and mutant peptides according to their MHC-I/HLA-I binding and TCR recognition types based on mutation positions within peptides and the influence of central tolerance, and further screening neoantigen peptides according to classification results.

The present invention relates to a novel method for predicting binding, transport, presentation, and TCR activation of wild-type/mutant peptides with MHC-I/HLA-I. Specifically, a model predictor with improved accuracy and biological interpretability is constructed by extracting protein residue combinations and key sites that play important roles during the binding process, better characterizing MHC-I/HLA-I sequences, and applying transfer learning techniques. Using the constructed model, key binding sites of MHC-I/HLA-I are explored, and neoantigens are further classified according to positions of mutations within peptides, thereby enhancing the accuracy of neoantigens identification. Compared with conventional classifiers, the method is more efficient and accurate, and has significant application value in tumor immunotherapy.

As demonstrated in the following embodiments, compared with other existing analysis methods, the present invention has the following advantages.

(1) Biological features inherent to protein sequences are applied in model construction. By learning the combination patterns of amino acid residues within protein sequences, high-frequency amino acid residue combinations and key sites with significant features are found, enabling construction of a tokenizer that provides a more rational representation of amino acid sequences.

(2) Natural language processing algorithms are applied to biological information language, namely protein sequences. That is, protein sequences are treated as sentences, and predictive models are constructed by using natural language processing methods to learn features and contextual relationships in sequences.

(3) Pre-trained models are constructed by using lots of existing unlabeled data and fine-tuned by using labeled data. By constructing a masked language model, the combination of masked amino acid residues can be inferred to learn contextual information and expression rules of protein sequences, enabling the pre-trained model to acquire rich language knowledge. Subsequently, fine-tuning the pre-trained model is performed by limited binging data of labeled peptides and MHC-I/HLA-I, thereby constructing a classification model more suitable for affinity prediction.

(4) By means of natural language processing models, attention scores for each position/motif within protein sequences can be output, thereby enabling determination of key sites in the binding of MHC-I/HLA-1 to peptide, determination of key binding sites of peptides, and derivation of more representative and significant characterization methods for MHC-I/HLA-I sequences.

(5) In addition to predicting the binding between peptides and MHC-I/HLA-I, features involving binding stability between peptides and MHC-I/HLA-I, transport, and presentation are also covered via concatenating. From the perspective of actual biological processes, features that play important roles in biological pathways are converted into numerical representations and combined with other features for model training and prediction, thereby improving the practical applicability of the model.

(6) By determining positions of tumor-specific mutations within peptides, peptides are classified based on different pMHC binding and TCR recognition patterns, thereby further screening out neoantigen peptides with more accuracy. According to literature reports, antigen sequences not only can bind to MHC, but also can be recognized by TCR. The positions of tumor-specific antigen mutation simultaneously affect MHC binding and TCR recognition. Therefore, key MHC binding sites are obtained based on attention scores at each antigen peptide position, and neoantigens are further classified according to mutation positions, thereby excluding peptides that exhibit strong MHC-I/HLA-I binding potential but fail to activate TCR, and improving the accuracy of antigen peptide screening. The improved algorithm enables more accurate prediction of whether wild-type/mutant peptides possess binding, transport, and presentation potential with major histocompatibility complex (MHC) and TCR recognition potential, thereby promoting identification of tumor neoantigens and their clinical application.

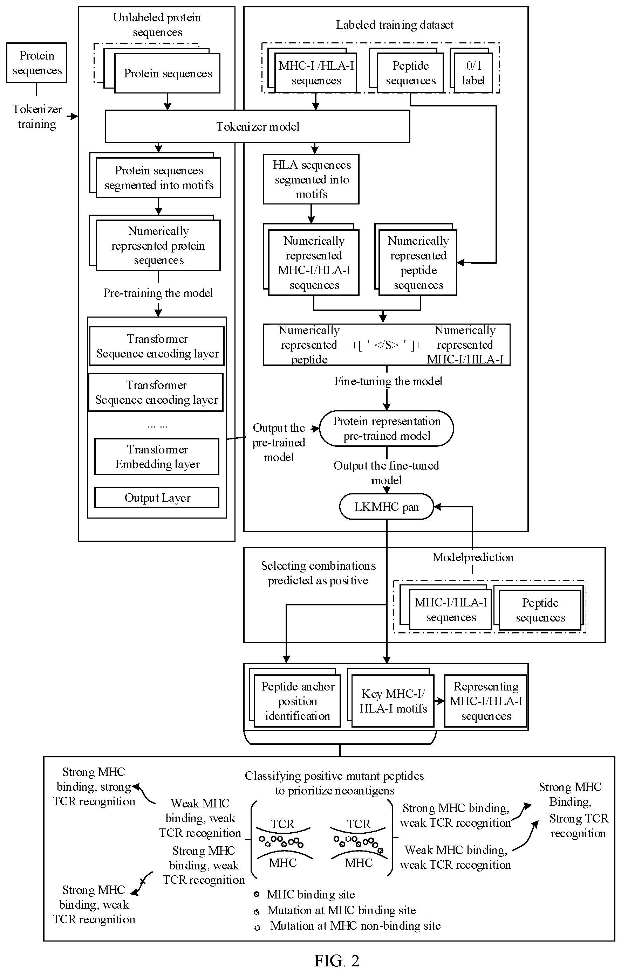

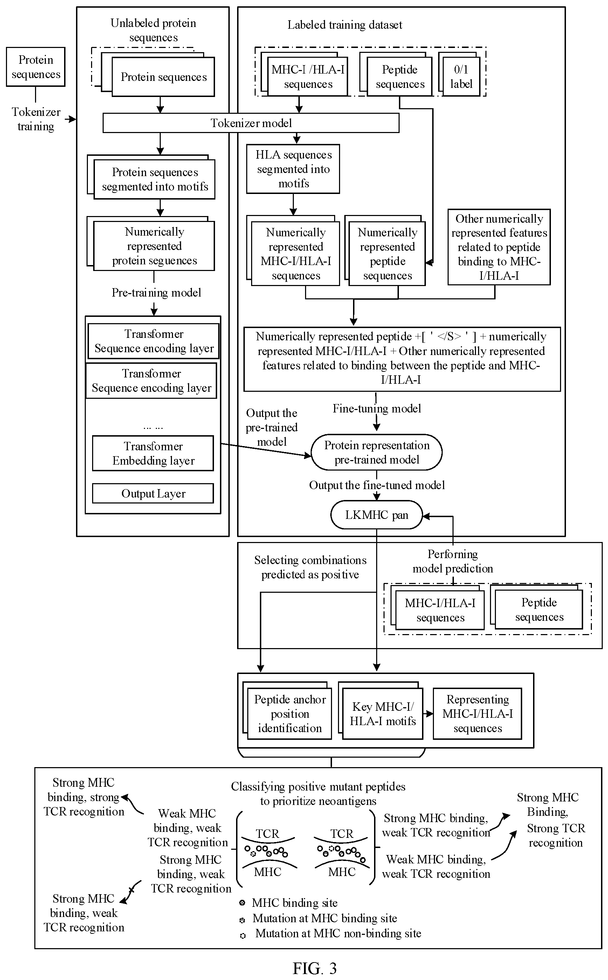

The following detailed description of the content of this application is provided with reference to FIG. 2 and FIG.3.

Embodiment 1

1.1 Dataset required for model training and screening on them
1.1.1 Obtaining protein sequences from databases such as UniProt;
1.1.2 Obtaining positive peptide sequences from databases such as IEDB, where the positive peptides must meet the following conditions:

The length thereof is 8-15 bp;
The MHC/HLA type for recognizing positive peptides is type I;
The corresponding wild-type peptide has a specific UniProt number.

1.1.3 Obtaining HLA sequences from databases such as IPD-IMGT/HLA, and obtaining HLA-I sequences after multiple sequence alignment for characterizing HLA sequences.
1.2 Constructing positive and negative datasets

The positive dataset consists of protein sequences/positive peptide sequences, in which the protein sequences are used to construct a pre-trained model and learn the expression features and contextual relationships. The positive peptide sequences are labeled peptides capable of binding to MHC-I/HLA-I. The negative sequences are randomly construct based on the positive sequences, meaning they are peptides that do not bind to MHC-I/HLA-I, deduced from positive peptides. The construction method for negative peptides is as follows:

Negative peptides with a length of 8-15 bp;
Randomly changing the amino acids within the same protein sequence as the positive peptide;
Removing peptides appearing in the positive peptide.

1.3 Constructing and applying a protein sequence tokenizer

1.3.1 amino acid residues are combined on a large number of proteins. By using machine learning algorithms or statistical inference models, so as to infer frequently occurring amino acid residue combinations in the protein sequence. These frequently occurring amino acid residue combinations are likely to be functional motifs, or in other words, play important roles when proteins perform their functions. Simultaneously, the locations of important motifs can be inferred. Unlike simply focusing on individual amino acid residues, the tokenizer is more helpful in exploring key features that enable proteins to perform their functions.

1.3.2 Protein sequence tokenization. All protein sequences are tokenized by using the tokenizer, and potentially functional amino acid residues are grouped together to facilitate subsequent model construction.

In this embodiment of the invention, the combination patterns of amino acid residues in protein sequences can be learn from a protein database, and high-frequency combinations of residues in the protein sequence are grouped into a group to construct a protein sequence tokenizer. Specifically, a protein dataset is download from a protein database to extract protein sequences therefrom; the protein sequences are trained based on the frequency of amino acid residues/residue combinations, left and right connectivity, and other characteristics of known protein sequences to obtain a tokenizer with interpretable amino acid residues of protein sequences.

1.4 Construction of protein representation pre-trained model

A large number of tokenized protein sequences imply protein function-related features, protein expression patterns, and relationships between motifs within the sequence. Therefore, a masked language model from natural language processing is used to randomly mask motifs in the protein sequences and thus predict the protein sequences, learning the features implied in the protein sequences. The masked language model is a self-supervised learning task designed to allow the model to learn the contextual information and language rules of motifs during pre-training. The model receives the input sequences, then randomly selects a certain ratio of motifs from the input text sequences to mask, and requires the model to predict these masked motifs. For example, following sentence are given:

Input sequence: MAF SAE DVL KE YD RRRR ME ALLL
Masked sequence: MAF SAE [mask] KE YD RRRR ME ALLL

In the present invention, unsupervised learning is performed on a large amount of unlabeled protein data, to learn rich motif-related information of proteins. Then, transfer learning techniques are applied to adapt these models to specific labeled classification tasks. Specifically, a large number of unlabeled protein sequences are input into a protein sequence tokenizer to tokenize the protein sequences. The protein sequences are represented in the form of amino acid residue combinations. A masked language pre-training model (MLM) is constructed to randomly mask amino acid residue combinations in the tokenized protein sequences. The contextual information and expression rules of the protein sequences are leant by the model when inferring the masked amino acid residue combinations, helping the model to get key residue features.

1.5 Fine-tuning the protein representation pre-trained model

1.5.1 The generated and labeled positive peptides capable of binding to MHC-I/HLA-I, and the negative peptides randomly generated based on the positive peptides, are labeled as 1 or 0, respectively. It is worth noting that these positive data are obtained through experiments, which means that only limited data for labeled training are available. This is the reason why a large amount of unlabeled protein data are used to create the pre-trained model first.
1.5.2 The labeled data in step 1.5.1 are divided into a training dataset/a validation dataset/a test dataset at a certain ratio.
1.5.3 Fine-tuning the generated and pre-trained model by using the data from step 1.5.2, and input them to construct a binary classification model.
1.5.4 The model structure is shown in FIG. 2.
1.6 Application of the binary classification model
1.6.1 Identifying peptides with the potential to bind to MHC-I/HLA-I.
1.6.2 Extracting key features including functional amino acid residue combinations motifs, important function sites/binding sites, etc from protein sequences.
Using the above methods, peptides which can predict the potential to bind to MHC-I/HLA-I can be constructed based on biological characteristics, and their key features can be identified.

1.7. Identifying the impact of mutation positions on the potential of peptide binding to MHC-I/HLA-I and TCR recognition

Based on the obtained peptides and MHC-I/HLA-I binding sites in combination with the location of mutations in wild-type/mutated peptides, and considering the influence of central tolerance mechanisms on neoantigen screening, pMHC binding and TCR recognition are categorized into four types in which the first three types have neoantigen potential, and the last type is required to be excluded upon neoantigen screening.
Category 1: The wild-type peptides exhibit weak binding to MHC-I/HLA-I and weak TCR recognition, while the mutant peptides have mutations at MHC-I/HLA-I binding sites and are strong in MHC-I/HLA-I binding and TCR recognition. Under the absence of central tolerance, mutations occurring at anchor positions are also capable of activating TCR.
Category 2: The wild-type peptides exhibit weak binding to MHC-I/HLA-I and weak TCR recognition, while the mutant peptides have mutations at a non-MHC-I/HLA-I binding site and are strong in MHC-I/HLA-I binding and TCR recognition. Under the absence of central tolerance, mutations occurring at non-anchor positions are capable of activating TCR.

Category 3: The wild-type peptides exhibit strong binding to MHC-I/HLA-I and weak TCR recognition, while the mutant peptides have mutations at a non-MHC-I/HLA-I binding site and are strong in MHC-I/HLA-I binding and TCR recognition. In the presence of central tolerance, mutations occurring at non-anchor positions are capable of activating TCR.

Category 4: The wild-type peptides exhibit strong binding to MHC-I/HLA-I and weak TCR recognition, while the mutant peptides have mutations at an MHC-I/HLA-I binding site and are strong in MHC-I/HLA-I binding and weak in TCR recognition. In the presence of central tolerance, mutations occurring at non-anchor positions are not capable of activating TCR. This can be further explained as follows: under this combination, the mutation sites are located at MHC-I/HLA-I binding sites, and the sequence exposed to the TCR recognition interface is not altered. As a result, such peptide is still affected by central immune tolerance and is not suitable as a neoantigen peptide.

Features that play important roles in peptide and MHC-I/HLA-I recognition are inferred in the present invention based on biological characteristics and attention scores, through the above steps including, representing MHC-I/HLA-I sequences and identifying key functional amino acid residue combinations and their positions; predicting, based on the binary classification model, the roles of amino acid residue combination types in the MHC-I/HLA-I sequence and their different positions in peptide recognition, binding, and presentation; and identifying key amino acid residue combinations at key sites according to attention scores; key binding sites of peptides are inferred to identify key functional amino acids and their positions. Using the obtained peptide binding sites information, wild-type/mutant peptides are classified with respect to MHC-I/HLA-I binding and TCR recognition based on the positions of mutations within the peptides. The influence of central tolerance on neoantigen screening is introduced, and neoantigen peptides are further excluded based on the classification results, thereby obtaining neoantigens with more accuracy.

Embodiment 2

In the present invention, protein sequences are obtained from the UniProt protein database to construct a tokenizer. Positive peptide binding sequences to MHC-I/HLA-I are obtained from the IEDB, and negative peptide are generated based on the positive sequences. Protein sequences tokenized by the tokenizer are used to construct a protein representation pre-trained model. Tokenized MHC-I/HLA-I sequences and peptides are converted into numerically represented formats and concatenated, and are input into the protein representation pre-trained model for fine-tuning, thereby ultimately obtaining a binary classification model capable of predicting peptide binding potential to MHC-I/HLA-I. Based on the trained model and algorithmic characteristics, key functional sites and motifs in protein sequences are extracted to represent MHC-I/HLA-I sequences; using the positions of tumor-specific mutations within antigenic peptides and their MHC binding potential, antigenic peptides are further classified, antigenic peptides that cannot be recognized by TCR due to the influence of central immune tolerance are excluded, and more accurate and comprehensive neoantigens are ultimately obtained. The specific steps include:

2.1 Construction and use of the tokenizer

A tokenizer is trained with a vocabulary size set to 10,000. The tokenization results are as follows:

Before tokenization: MIINPTSDPEVSALEKKNTGRIAQIIGPVLDVTFPPGKMP......
After tokenization: MII NP TTSD PE VS ALEK KN TGRI AQI IGP VLD VT FP PG KMP......

2.2 Construction of the Pre-trained Model

A natural language model is used to construct a protein representation pre-trained model. Unsupervised training is performed on tokenized protein sequences to learn linguistic characteristics and contextual relationships of protein sequences. A masking ratio is set to 15%. A total of 568,745 protein sequences are used, with a total sentencepiece size of 206M.

2.3 Fine-tuning the protein representation pre-trained model

2.3.1 MHC-I/HLA-I sequences are tokenized according to the corresponding negative peptide generated by the positive peptide binging to MHC-I/HLA-I in which the data for the positive or negative peptides are labeled as 1 or 0, respectively. The positive dataset is distributed as follows

Table 1

| Number of positive peptides | Number of negative peptides | Number of HLA types |
|---|---|---|
| 502,735 | 12,505,198 | 121 |

As shown in Table 1, there are only 121 HLA types. Therefore, performing protein feature identification by using such a limited dataset is highly challenging, which constitutes the reason for first constructing a protein representation pre-trained model.

2.3.2 Example of raw data format input to the protein representation pre-trained model

E I E I C D G F,M RVT AP RTLL LLL WG AV ALT ET WAG SH SM RY FH TS VS RPG RG EP RFI TVG YVD DT…….AGL AV ------ L AVV - VIG AV VAA VM - CRR KS SGG KGG SY SQ AAC SD S AQG SD VSL TA -----------,0

2.4 Application of the binary classification model

2.4.1 By inputting data into the binary classification model in accordance with the data format described in Section 2.3.2, the binding potential between peptides and MHC-I/HLA-I can be predicted, and the prediction results are shown in Table 2.

Table 2

| peptide | MHC | Label | prediction | probability |
|---|---|---|---|---|
| KAISKNVLF | MRVT......QTA------------ | 1 | 1 | [0.050, 0.950] |
| RPTRPDKARR | MAV......CKV--------- | 0 | 0 | [0.990,0.001] |

where "Label" denotes a ground-truth label, "Prediction" denotes a label predicted by the model, and "probability" denotes a model-predicted result [A,B] in which A represents a probability that the prediction result is negative, and B represents a probability that the prediction result is positive.

2.4.2 Evaluation of the binary classification model

The evaluation dataset is a validation dataset that is independent of the dataset used for model training. The composition of the validation dataset is shown in Table 3.

Table 3

| Number of positive peptides | Number of negative peptides | Number of HLA types |
|---|---|---|
| 3,725 | 108,113 | 40 |

Model performance is calculated by using the following formulas:

$$Precision = \frac{TP}{TP + FP}$$

$$Recall/TPR = \frac{TP}{TP + FN}$$

$$FPR = \frac{FP}{TN + FP}$$

TP: true positives; TN: true negatives; FP: false positives; FN: false negatives;

AUC is the area under the curve of the true positive rate (TPR) versus the false positive rate (FPR).

A comparison with the commonly used NetMHCpan 4.1 software is performed to evaluate model performance, and the results are shown in Table 4.

Table 4

| Type | NetMHCpan4.1 | LKMHCpan |
|---|---|---|
| AUC | 0.77 | 0.85 |
| Precision | 0.75 | 0.84 |
| Recall | 0.79 | 0.85 |

2.4.3 A method is provided for extracting MHC-I/HLA-I binding sites and obtaining positions of key functional amino acid residues, so as to biologically interpret the prediction performance of the model. Heat maps are depicted based on attention scores at different positions, in which brighter colors indicate a higher likelihood of being key binding sites between peptides and MHC. This also indirectly demonstrates the favorable performance of the model, and thus it can be further used for extracting key site features of MHC-I/HLA-I sequences in subsequent steps.

2.4.4 A method is provided for representing protein sequences and identifying key functional amino acid residue combinations and their positions, so as to biologically interpret the prediction performance of the model. Heat maps are depicted based attention scores at each position along HLA-A/B/C sequences, to infer overall key binding sites of MHC-I/HLA-I sequences, thereby facilitating improved representation of MHC-I/HLA-I sequences. Compared with hotspot positions provided by common LKMHCpan or MHCflurry, (31, 33, 48, 69, 83, 86, 87, 90, 91, 93, 94, 97, 98, 100, 101, 104, 105, 108, 115, 119, 121, 123, 126, 138, 140, 142, 167, 171, 174, 176, 180, 182, 183, 187, 191, 195, and 223) are more comprehensive and more representative. When focusing on functional motif sequences at hotspots, and further extracting additional key sites beyond the above positions, such as positions 39 and 235, it would be more consistent with the functional significance embedded in protein sequences.

2.5 Adding classification information for peptide binding to MHC-I/HLA-1 and TCR recognition to promote more detailed and comprehensive neoantigen sequence identification.

Based on the above analysis, after obtaining the anchor positions for peptide binding to MHC-I/HLA-I, peptide types can be classified into four groups according to the binding potential of wild-type peptides/mutant peptides and the positions of tumor-specific mutations, as shown in Table 5.

Table 5

| Category | Mutation Position | Wild-Type Peptide | Mutant Peptide | Suitable for Selection as Neoantigen |
|---|---|---|---|---|
| 1 | MHC binding site | Weak MHC binding & weak TCR recognition | Strong MHC binding & strong TCR recognition | Yes |
| 2 | Non-MHC binding site | Weak MHC binding & weak TCR recognition | Strong MHC binding & strong TCR recognition | Yes |
| 3 | Non-MHC binding site | Strong MHC binding & weak TCR recognition | Strong MHC binding & strong TCR recognition | Yes |
| 4 | MHC binding site | Strong MHC binding & weak TCR recognition | Strong MHC binding & weak TCR recognition | No |

Antigen peptides belonging to Category 4 are excluded during neoantigen screening, thereby improving the accuracy of neoantigen prediction.

Embodiment 3

Steps 3.1 to 3.4 are the same as steps 1.1 to 1.4 in Embodiment 1.

3.5 Fine-tuning the protein representation pre-trained model

3.5.1 The generated and labeled positive peptides that can bind to MHC-I/HLA-I, and the negative peptides randomly generated based on the positive peptides, are labeled as 1 or 0, respectively. It is worth noting that these positive data are obtained through experiments, which means that only a limited data for labeled training data are available. This is the reason why a large amount of unlabeled protein data are used to create the pre-trained model first.

3.5.2 The labeled MHC-I/HLA-I sequences and peptide sequences tokenized by the protein tokenizer in step 3.5.1 are converted into numerical representations and concatenated. The concatenation strategy is as follows.

The probability of cleavage of the protein sequence is calculated based on the peptide and its corresponding protein sequence.

The binding stability between the peptide and MHC-I/HLA-I, as well as the presentation capability after binding are calculated.

The numerically represented probability of cleavage of the protein sequence, the binding stability between the peptide and MHC-I/HLA-I, and the presentation capability following binding between the peptide and MHC-I/HLA-I are concatenated with the numerically represented MHC-I/HLA-I sequences and peptide sequences tokenized by the protein tokenizer.

3.5.3 The labeled data in step 3.5.2 are divided into a training dataset/a validation dataset/a test dataset at a certain ratio.

3.5.4 Fine-tuning the generated and pre-trained model by using the data from step 3.5.3, and input them to construct a binary classification model.

3.5.5 The model structure is shown in FIG. 3.

Steps 3.6 to 3.7 are the same as steps 1.6 to 1.7 in Embodiment 1.

Features that play important roles in peptide and MHC-I/HLA-I recognition are inferred in the present invention based on biological characteristics and attention scores through representing MHC-I/HLA-I sequences and identifying key functional amino acid residue combinations and their positions; predicting, based on the binary classification model, the roles of amino acid residue combination types in the MHC-I/HLA-I sequence and their different positions in peptide recognition, binding, and presentation; and identifying key amino acid residue combinations at key sites according to attention scores; key binding sites of peptides are inferred to identify key functional amino acids and their positions. Using the obtained peptide binding sites information, wild-type/mutant peptides are classified with respect to MHC-I/HLA-I binding and TCR recognition based on the positions of mutations within the peptides. The influence of pMHC binding stability, pMHC presentation capability, and central tolerance on neoantigen screening are introduced, and neoantigen peptides are further excluded based on the classification results, thereby obtaining neoantigens with more accuracy.

Embodiment 4

In the present invention, protein sequences are obtained from the UniProt protein database to construct a tokenizer. Positive peptide binding sequences to MHC-I/HLA-I are obtained from the IEDB, and negative peptide are generated based on the positive sequences. Protein sequences tokenized by the tokenizer are used to construct a protein representation pre-trained model. Tokenized MHC-I/HLA-I sequences and peptides are converted into numerically represented formats. Probability of cleavage of protein sequences is calculated based on peptides and their corresponding protein sequences, binding stability between peptides and MHC-I/HLA-I, as well as presentation likelihood following binding, are calculated based on peptides and MHC-I/HLA-I. The numerically represented MHC-I/HLA-I sequences and peptide sequences are concatenated together with the corresponding probability of cleavage of peptide, pMHC binding stability, and pMHC presentation likelihoods by torch.cat() as the concatenating manner, and are input into the protein representation pre-trained model for fine-tuning. The original pre-trained model is inherited while adding a fully connected layer and a classification layer, thereby ultimately obtaining a binary classification model capable of predicting the binding potential between peptides and MHC-I/HLA-I. Based on the trained model and algorithmic characteristics, key functional sites and motifs in protein sequences are extracted to represent MHC-I/HLA-I sequences. using the positions of tumor-specific mutations within antigenic peptides and their MHC binding potential, antigenic peptides are further classified, antigenic peptides that cannot be recognized by TCR due to the influence of central immune tolerance are excluded, and more accurate and comprehensive neoantigens are ultimately obtained. The specific steps include:

Steps 4.1 to 4.2 are the same as steps 2.1 to 2.2 in Embodiment 2.

4.3 Fine-tuning the protein representation pre-trained model

4.3.1 MHC-I/HLA-I sequences are tokenized according to the corresponding negative peptide generated by the positive peptide binging to MHC-I/HLA-I in which the data for the positive or negative peptides are labeled as 1 or 0, respectively. The positive dataset is distributed as shown in Table 6.

Table 6

| Number of positive peptides | Number of negative peptides | Number of HLA types |
|---|---|---|
| 502,735 | 12,505,198 | 121 |

4.3.2 Features that influence binding between peptides and MHC-I/HLA-I are calculated, including probability of cleavage of peptide, pMHC stability, and presentation likelihood of pMHC after binding. For example, the calculation mentioned may be performed as follows:

By using software pepsickle-v0-2-2, peptide sequences are input to output probability of cleavage scores at each position. The probability of cleavage of peptide is calculated as the average of cleavage scores of the C-terminal/N-terminal amino acids of the peptide, with a value range of [0, 1], in which higher score indicates a higher probability of cleavage.

By using the software LKMHCstablepan-v1, peptide sequences and MHC-I/HLA-I sequences are input to calculate pMHC binding stability, and a pMHC binding stability score is output, in which higher score indicates greater stability;

By using the software DeepTAP-v1.0.1, peptide sequences are input to calculate the likelihood of presentation to the cell surface following pMHC binding, and a presentation likelihood score is output with a value range of [0, 1], in which a higher score indicates a higher presentation likelihood.

4.3.3 Example of the raw data format input into the protein representation pre-trained model is as follows:
E I E I C D G F,M RVT AP RTLL LLL WG AV ALT ET WAG SH SM RY FH TS VS RPG RG EP RFI TVG YVD DT.......AGL AV ------ L AVV - VIG AV VAA VM - CRR KS SGG KGG SY SQ AAC SD S AQG SD VSL TA ------------,0.057,0.20,0.12,0 (Corresponding in order to: peptide sequence, MHC-I/HLA-I sequence, probability of cleavage of peptide, score of pMHC binding stability, score of presentation likelihood of pMHC after binding, positive or negative).

After the MHC-I/HLA-I sequences and peptide sequences tokenized by the tokenizer are converted into numerically represented sequences, they are concatenated, along a specified dimension, with the probability of cleavage score of a peptide, the pMHC binding stability score, and the pMHC presentation likelihood score, and then input into the protein representation pre-trained model for model training.

4.4 Application of the binary classification model

4.4.1 By inputting data into the binary classification model in accordance with the data format described in Section 4.3.3, the binding potential between peptides and MHC-I/HLA-I can be predicted, and the prediction results are shown in Table 7.

Table 7

| Peptide | MHC | Label | Prediction | Probability |
|---|---|---|---|---|
| KAISKNVLF | MRVT...... QTA------------ | 1 | 1 | [0.0320, 0.9681] |
| RPTRPDKARR | MAV......CKV------- | 0 | 0 | [0.9995,0.0005] |

where "Label" denotes a ground-truth label, "Prediction" denotes a label predicted by the model, and "probability" denotes a model-predicted result [A,B] in which A represents a probability that the prediction result is negative, and B represents a probability that the prediction result is positive.

4.4.2 Evaluation of the binary classification model

The evaluation dataset is the same as that in step 2.4.2 of Embodiment 2.

The formula for calculating the model performance is the same as that in step 2.4.2 of Embodiment 2.

Compared with the commonly used NetMHCpan 4.1 software and the binary classification model constructed in Embodiment 2, model performance evaluation is conducted, as shown in Table 8:

Table 8

| Type | NetMHCpan4.1 | LKMHCpan(Embodiment 2) | LKMHCpan(Embodiment 4) |
|---|---|---|---|
| AUC | 0.77 | 0.85 | 0.89 |
| Precision | 0.75 | 0.84 | 0.91 |

(continued)

| Type | NetMHCpan4.1 | LKMHCpan(Embodiment 2) | LKMHCpan(Embodiment 4) |
|---|---|---|---|
| Recall | 0.79 | 0.85 | 0.86 |

Steps 4.4.3 to 4.4.4 are the same as steps 2.4.3 to 2.4.4 in Embodiment 2.

Step 4.5 is the same as step 2.5 in Embodiment 2.

The preferred embodiments of the present invention have been described above with reference to the accompanying drawings, and the present invention is not limited thereto. Any modifications, equivalent substitutions, and improvements made by those skilled in the art without departing from the scope and spirit of the present invention shall fall within the scope of protection of the present invention.

**Claims**

1. A method for identifying peptides capable of binding to MHC- I/HLA- I and being recognized by T-cell receptor (TCR), comprising:

   acquiring protein sequences, major histocompatibility complex class I (MHC I) / major histocompatibility complex class I in humans (HLA- I) sequences, and peptide sequences;
   training a protein sequence tokenizer by using the protein sequences;
   training a protein representation model by using the protein sequences tokenized by the protein sequence tokenizer to obtain a trained protein representation pre-trained model;
   fine-tuning the trained protein representation pre-trained model by using the MHC- I/HLA- I sequences and the peptide sequences tokenized by the protein sequence tokenizer, to obtain a binary classification model; and
   identifying target wild-type/mutant peptides and classifying mutant peptides, by using the binary classification model, to obtain candidate neoantigen mutant peptides that are positively correlated with TCR recognition;
   wherein, the MHC-I refers to major histocompatibility complex class I; the HLA-I refers to major histocompatibility complex class I in humans; and the TCR refers to a T cell receptor.

2. The method according to claim 1, wherein the peptide sequences comprise positive peptide sequences and negative peptide sequences, wherein the positive peptide sequences are peptides capable of binding to MHC- I/HLA- I and are labeled as 1, and the negative peptide sequences are peptide sequences randomly generated from protein sequences based on features of the positive peptide sequences and are labeled as 0.

3. The method according to claim 2, wherein the protein sequences comprise protein sequences collected from a protein database; accordingly, collected protein sequences are used to train a tokenizer with high-frequency repeated amino acid residue combination motifs, to obtain the protein sequence tokenizer.

4. The method according to claim 3, wherein the step of training the protein representation model by using the protein sequences tokenized by the protein sequence tokenizer to obtain the trained protein representation pre-trained model comprises:

   inputting unlabeled protein sequences into the protein sequence tokenizer for tokenization, to obtain protein sequences segmented into functional amino acid residue combination motifs;
   converting the protein sequences segmented into the functional amino acid residue combination motifs into numerically represented protein sequences; and
   training the protein representation model by using the numerically represented protein sequences to obtain the trained protein representation pre-trained model.

5. The method according to claim 4, wherein the step of fine-tuning the trained protein representation pre-trained model by using the MHC- I/HLA- I sequences and the peptide sequences that have been tokenized by the protein sequence tokenizer to obtain the binary classification model comprises:

   inputting labeled MHC- I/HLA- I sequences into the protein sequence tokenizer for tokenization, to obtain MHC- I/HLA- I sequences segmented into functional amino acid residue combination motifs, and converting the MHC- I/HLA- I sequences segmented into functional amino acid residue combination motifs into numerically represented MHC- I/HLA- I sequences;

converting labeled peptide sequences into numerically represented peptide sequences; and

concatenating the numerically represented MHC- I/HLA- I sequences and the numerically represented peptide sequences to obtain concatenated sequences, and fine-tuning the trained protein representation pre-trained model by using the concatenated sequences to obtain the binary classification model.

6. The method according to claim 5, wherein the concatenating further comprises concatenating the numerically represented MHC-I/HLA-I sequences, the numerically represented peptide sequences, and numerically represented additional features related to binding between the peptides and the MHC-I/HLA-I, wherein the additional features are one or more selected from a probability of cleavage of the protein sequences, a binding stability between the MHC-I/HLA-I and the peptides, and a presentation likelihood of complexes formed by the MHC-I/HLA-I and the peptides.

7. The method according to claim 6, wherein prior to the fine-tuning, the method further comprises calculating the additional features related to binding between the peptides and the MHC-I/HLA-I, by using the protein sequences, the MHC-I/HLA-I sequences, and the peptide sequences.

8. The method according to claim 5, wherein the step of identifying target wild-type/mutant peptides by using the binary classification model comprises:

inputting the target wild-type/mutant peptides into the binary classification model, and outputting attention scores for each position of the mutant peptides predicted as positive, and attention scores of functional amino acid residue combination motifs of the MHC- I/HLA- I sequences; and

determining anchor positions of binding between the MHC- I/HLA- I sequences and the positive peptides, as well as key representative positions of the MHC- I/HLA- I sequences, based on the attention scores for each position of the mutant peptides predicted as positive and the attention scores of the MHC- I/HLA- I sequences motifs, and classifying positive mutant peptides based on the anchor positions of binding between the MHC- I/HLA- I sequences and the peptides and mutation positions of the peptides.

9. The method according to claim 8, wherein the classifying based on the anchor positions of binding between the MHC-I/HLA- I sequences and the positive mutant peptides as well as mutation positions relative to wild-type peptides comprises:

classifying the positive mutant peptides based on the anchor positions of binding between the MHC- I/HLA- I sequences and the peptides in combination with the mutation positions, and determining, based on classification results, whether the positive mutant peptides are suitable for selection as candidate neoantigen mutant peptides that are positively correlated with TCR recognition.

10. The method according to claim 9, wherein the determining, based on the classification results, whether the positive mutant peptides are suitable for selection as candidate neoantigen mutant peptides that are positively correlated with TCR recognition comprises:

determining that the peptides are selectable as neoantigen mutant peptides, when the wild-type peptides exhibit weak binding to MHC- I/HLA- I and weak TCR recognition, and the mutant peptides have mutations at anchor positions for MHC-I/HLA-I and are strong in MHC- I/HLA- I binding and TCR recognition;

determining that the peptides are selectable as neoantigen mutant peptides, when the wild-type peptides exhibit weak binding to MHC- I/HLA- I and weak TCR recognition, and the mutant peptides have mutations at non-anchor positions for MHC- I/HLA- I and are strong in MHC- I/HLA- I binding and TCR recognition;

determining that the peptides are selectable as neoantigen mutant peptides, when the wild-type peptides exhibit strong binding to MHC-I/HLA-I and weak TCR recognition, and the mutant peptides have mutations at non-anchor positions for MHC- I/HLA- I and are strong in MHC- I/HLA- I binding and strong TCR recognition;

determining that the peptides are not selectable as neoantigen mutant peptides, when the wild-type peptides exhibit strong binding to MHC- I/HLA- I and weak TCR recognition, and the mutant peptides have mutations at anchor positions for MHC- I/HLA- I and are strong in MHC- I/HLA- I binding and weak in TCR recognition.

11. An electronic device, comprising: a memory; a processor; and a computer program, wherein the computer program is stored in the memory and configured to be executed by the processor to implement the method according to any one of claims 1-10.

12. A computer-readable storage medium, having a computer program stored thereon, wherein the computer program is executed by a processor to implement the method according to any one of claims 1- 10.

| | S101 |
| Acquiring protein sequences, MHC-I/HLA-I sequences, and peptide sequences | |

| | S102 |
| Training a protein sequence tokenizer using the protein sequences | |

| | S103 |
| Training a protein representation model using the protein sequences tokenized by the protein sequence tokenizer to obtain a trained protein representation pre-trained model | |

| | S104 |
| Fine-tuning the trained protein representation pre-trained model by concatenating the MHC-I/HLA-I sequences and the peptide sequences tokenized by the protein sequence tokenizer to obtain a binary classification model | |

| | S105 |
| Identifying target wild-type/mutant peptides and classifying the mutant peptides using the binary classification model to obtain candidate neoantigen mutant peptides that are positively correlated with TCR recognition | |

FIG. 1

FIG. 2

FIG. 3

## INTERNATIONAL SEARCH REPORT

| | |
|---|---|
| | International application No. |
| | **PCT/CN2024/120794** |

**A.    CLASSIFICATION OF SUBJECT MATTER**

G16B30/00(2019.01)i;  G16B40/20(2019.01)i;  G06F16/33(2025.01)i;  G06F16/35(2025.01)i;  G06F18/24(2023.01)i;  G06F40/289(2020.01)i

According to International Patent Classification (IPC) or to both national classification and IPC

**B.    FIELDS SEARCHED**

Minimum documentation searched (classification system followed by classification symbols)

IPC: G16B, G06F

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, 万方数据, WANFANG DATA, Web of Science, BING, 百度学术, BAIDU SCHOLAR, incoPat, 中国生物序列检索系统, China Patents Biological Sequence Search System, NCBI, EBI, STNext: 申请人, 发明人, MHC, 主要组织相容性复合物I, HLA, 人白细胞抗原, human leukocyte antigen, TCR, T细胞受体, 肽段, peptide fragment, 分词, 分割, segment, 拆分, split, 切分, cut, 训练, training, 模型, model, 序列1-6

**C.    DOCUMENTS CONSIDERED TO BE RELEVANT**

| Category* | Citation of document, with indication, where appropriate, of the relevant passages | Relevant to claim No. |
|---|---|---|
| A | WO 2020132235 A1 (MERCK SHARP & DOHME CORP.) 25 June 2020 (2020-06-25) claims 1-37 | 1-12 |
| A | CN 112614538 A (XIAMEN UNIVERSITY) 06 April 2021 (2021-04-06) claims 1-8 | 1-12 |
| A | CN 110752041 A (SHENZHEN YUCE BIOTECHNOLOGY CO., LTD.) 04 February 2020 (2020-02-04) claims 1-10 | 1-12 |
| A | CN 1773517 A (SHANGHAI JIAO TONG UNIVERSITY) 17 May 2006 (2006-05-17) claims 1-8 | 1-12 |
| A | CN 116368570 A (MYNEO N.V.) 30 June 2023 (2023-06-30) claims 1-17 | 1-12 |

☑ Further documents are listed in the continuation of Box C.       ☑ See patent family annex.

| | |
|---|---|
| *    Special categories of cited documents: | "T"   later document published after the international filing date or priority date and not in conflict with the application but cited to understand the principle or theory underlying the invention |
| "A"  document defining the general state of the art which is not considered to be of particular relevance | |
| "D"  document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"  document referring to an oral disclosure, use, exhibition or other means | "&"   document member of the same patent family |
| "P"   document published prior to the international filing date but later than the priority date claimed | |

| Date of the actual completion of the international search | Date of mailing of the international search report |
|---|---|
| **07 January 2025** | **13 January 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/120794** |

| C. | DOCUMENTS CONSIDERED TO BE RELEVANT | |
| --- | --- | --- |
| Category* | Citation of document, with indication, where appropriate, of the relevant passages | Relevant to claim No. |
| A | 王广志 (WANG, Guangzhi). "基于个性化肿瘤HLA-I类新生抗原肽预测模型的改进研究 (Non-official translation: Research on the Improvement of Predictive Models for Personalized Tumor HLA-I Neoantigen Peptides)" 中国优秀硕士学位论文全文数据库工程科技I辑 *(Engineering Science & Technology I, China Master's Theses Full-text Database)*, No. 2, 15 February 2021 (2021-02-15), number B016-2748 abstract | 1-12 |
| PX | CN 116994654 A (BEIJING LIKANG LIFE TECHNOLOGY CO., LTD.) 03 November 2023 (2023-11-03) claims 1-10 | 1-12 |

Form PCT/ISA/210 (second sheet) (July 2022)

## INTERNATIONAL SEARCH REPORT

| International application No. |
|---|
| **PCT/CN2024/120794** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑   forming part of the international application as filed.

     b.    ☐   furnished subsequent to the international filing date for the purposes of international search (Rule 13*ter*.1(a)),

           ☐   accompanied by a statement to the effect that the sequence listing does not go beyond the disclosure in the international application as filed.

2. ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT
Information on patent family members

International application No.

**PCT/CN2024/120794**

| Patent document cited in search report | | | Publication date (day/month/year) | Patent family member(s) | | | Publication date (day/month/year) |
|---|---|---|---|---|---|---|---|
| WO | 2020132235 | A1 | 25 June 2020 | US | 2022076783 | A1 | 10 March 2022 |
| CN | 112614538 | A | 06 April 2021 | None | | | |
| CN | 110752041 | A | 04 February 2020 | None | | | |
| CN | 1773517 | A | 17 May 2006 | None | | | |
| CN | 116368570 | A | 30 June 2023 | EP | 4229640 | A1 | 23 August 2023 |
| | | | | EP | 4229640 | B1 | 04 December 2024 |
| | | | | CA | 3198427 | A1 | 21 April 2022 |
| | | | | US | 2024274231 | A1 | 15 August 2024 |
| | | | | WO | 2022079255 | A1 | 21 April 2022 |
| CN | 116994654 | A | 03 November 2023 | None | | | |

Form PCT/ISA/210 (patent family annex) (July 2022)